Europäisches Patentamt

European Patent Office

Office européen des brevets

(19)

(11) Publication number: **0 376 851**

**A2**

# EUROPEAN PATENT APPLICATION

(12)

(21) Application number: 89403672.2

(22) Date of filing: 28.12.89

(51) Int. Cl.5: **C12Q 1/02, G01N 33/50, G01N 33/53, //C12P21/08, C12Q1/68**

(30) Priority: 29.12.88 US 291730

(43) Date of publication of application:
04.07.90 Bulletin 90/27

(84) Designated Contracting States:
AT BE CH DE ES FR GB GR IT LI LU NL SE

(71) Applicant: CYTOGEN CORPORATION
201 College Road East Princeton Forrestal
Center
Princeton New Jersey 08540(US)

(72) Inventor: **Rodwell, John D.**
430 Ramsey Road
Yardley, PA 19067(US)
Inventor: **McKearn, Thomas J.**
RD No. 3, Box 119
New Hope, PA 18938(US)

(74) Representative: **Warcoin, Jacques et al**
Cabinet Régimbeau 26, avenue Kléber
F-75116 Paris(FR)

(54) **Molecular recognition units.**

(57) A system or method for identifying and/or designing novel peptides and polypeptides comprising an amino acid sequence which mimics the molecular recognition site of either (a) a macromolecule such as an immunoglobulin, an enzyme, a receptor protein, a lectin or other binding protein or (b) a small molecule or a small region of a large molecule which functions as a ligand and is recognized and binds specifically to a macromolecule is disclosed. Novel peptides and polypeptides as well as conjugates of the peptides and polypeptides are also disclosed. Applications for use of the peptides, polypeptides and conjugates in a wide range of fields such as biomedicine; biological control and pest regulation; agriculture; cosmetics; environmental control and waste management; chemistry; catalysis; nutrition and food industries; military uses; climate control, etc. are disclosed.

# MOLECULAR RECOGNITION UNITS

## 1. FIELD OF THE INVENTION

The present invention relates to a system or method for identifying and/or designing peptides and polypeptides which mimic the binding specificity of a number of naturally occurring macromolecules which recognize and bind specifically to small molecules or small regions of large molecules. The invention further relates to a method for designing peptides and polypeptides which mimic the molecular topography of small molecules or small regions of large molecules which function as ligands and are recognized by and bind specifically to the naturally occurring macromolecules.

More particularly, the invention encompasses a method for identifying and/or designing novel peptides and polypeptides which comprise an amino acid sequence which mimics the molecular recognition site, and hence the binding specificity, of macromolecules including, but not limited to: immunoglobulins or antibodies, enzymes, receptor proteins, lectins and other binding proteins such as avidin and streptavidin, etc. Additionally, the invention encompasses a method for designing novel peptides and polypeptides which comprise an amino acid sequence which mimics the molecular recognition site of small molecules or small regions of large molecules which function as ligands and are recognized by and bind specifically to a wide range of naturally occurring macromolecules. Examples of molecules which function as ligands include, but are not limited to antigens; hormones, pheromones, neurotransmitters, signal proteins and peptides, prostaglandins, etc.

The invention further encompasses the novel peptides and polypeptides prepared according to the method or system described herein. Also encompassed are novel conjugates in which a peptide or polypeptide of the invention is attached, either directly or via a linker moiety, to a second compound. The conjugates, include, but are not limited to fusion proteins containing the novel peptides and polypeptides.

In addition, the invention encompasses the nucleotide sequences which encode the novel peptides and polypeptides prepared by the method described.

The novel peptides, polypeptides and conjugates of the invention are advantageously used for a wide range of applications including uses in the fields of biomedicine; biological control and pest regulation; agriculture; cosmetics; environmental control and waste management; chemistry; catalysis; nutrition and food industries; military uses; and climate control.

## 2. BACKGROUND OF THE INVENTION

Molecular recognition, i.e. the ability to specifically recognize and bind another molecule such as a ligand or a substrate is an important functional property of a number of naturally occurring macromolecules which are critically important to biological and/or biochemical systems. Formation of the macromolecule/ligand complex leads to additional processes depending upon the nature of the particular macromolecule and, at times, upon the environment in which the complex forms. For instance, recognition and binding of an enzyme to its substrate leads to chemical changes in that substrate; recognition and binding of an antibody or immunoglobulin to its antigen, to immobilization and/or inactivation of such antigen; recognition and binding of a carrier or transport protein to its ligand, to transport of the ligand from one site to another; recognition and binding of a hormone receptor to its hormone, to changes in cellular activity; recognition and binding of a contractile protein to its ligand, to mechanical work; etc. Thus, these naturally occurring macromolecules and their ligands are a critical component of living systems and are particularly useful in biology and medicine for delivery of agents to target sites, for diagnosis, therapy, bioregulation, separations and preparations, etc. In addition, macromolecules and their ligands find uses in numerous non-medical, non-biological, industrial applications such as separations, preparations, catalysis and monitoring of chemicals and other substances.

Efficient and inexpensive methods for preparing homogenous macromolecules and/or ligands for such macromolecules in large quantities are highly desired. The development of monoclonal antibody techniques has provided a fairly efficient means to obtain homogenous supplies of one category of macromolecules, i.e. antibodies or immunoglobulins. Recombinant DNA technology has also provided the means to obtain homogenous supplies of some proteinaceous macromolecules and ligands. Chemical synthetic methods can provide a means for preparing some macromolecules and ligands, provided knowledge of the structure and chemical composition of such substances is available.

Despite the above mentioned techniques, there still exists a very real need for efficient, inexpensive methods for preparing large, homogeneous amounts of macromolecules and their ligands, whether proteinaceous or not, for biomedical and industrial applications. Moreover, in certain instances, it is desired to prepare substances having the molecular recognition and binding specificity of a naturally occurring macromolecule without additional effector activity of such molecule. To illustrate, it may be desired to obtain a molecule having the specific binding activity of a given antibody without the additional activity of such antibody such as complement-mediated cell lysis or hypersensitive or allergic reactions mediated by binding of the Fc region of the antibody to cell surface receptors. Thus, there exists a need for an efficient method to prepare molecules that can mimic the binding specificity of either a naturally occurring macromolecule or a ligand which is recognized by the naturally occurring macromolecule.

A method for preparing large, single polypeptide chains which are stated to have the characteristics and binding specificity of the antigen binding region of antibodies is described by Ladner (International Pat. Application No. PCT/US87/02208 published on March 10, 1988; see also United States Pat. No. 4704692 issued on November 3, 1987). According to this method, one or more peptide linkers are devised using a computer-based system and are used to join the variable regions of an antibody to form a single polypeptide chain of approximately 215-250 amino acid residues which exhibits three-dimensional topography similar to that of the original antibody. The nucleotide sequence encoding such large polypeptide is deduced, synthesized and inserted using recombinant DNA techniques into a suitable host cell which expresses the desired polypeptide.

Unlike the method described by Ladner which provides large polypeptide molecules of about 215-250 amino acid residues, the presently claimed method provides small peptides and polypeptides of less than about 40-45 amino acid residues which mimic the binding specificity of macromolecules and ligands. In addition, the present method does not require the preparation and synthesis of linkers which are necessary according to Ladner to enable the large polypeptide chains to mimic the binding of an antibody molecule. Moreover, although recombinant DNA techniques can be employed to prepare the presently claimed peptides, polypeptides and conjugates, unlike the method of Ladner, the present method does not require such techniques. Less expensive, efficient chemical synthetic methods can advantageously be used to prepare the presently claimed compositions.

Bruck et al. (1986, Proc. Nat'l Acad. Sci. USA 83:6578-82) describes the molecular cloning and nucleic acid sequence of the heavy and light variable regions ($V_H$ and $V_L$) of an anti-idiotypic monoclonal antibody, 87.92.6, which is stated to antigenically and functionally mimic the receptor binding structure of the retrovirus type 3 hemagglutinin (HA). Bruck et al. state that their results suggest that molecular mimicry of the viral HA glycoprotein by the anti-idiotypic antibody may be created by a stretch of homologous amino acids on the antibody $V_L$. The authors conclude that proteinaceous antigen mimicry by antibodies is achieved by sharing primary structure, i.e. homologous amino acid sequences found on the antigen and antibody.

Unlike the disclosure of Bruck et al., the method of the present invention allows the generation of small peptides and polypeptides having the binding · specificity of a macromolecule or a ligand in the absence of knowledge of the primary structure of the macromolecule or ligand intended to be mimicked. Moreover, the present method can be employed to prepare peptides and polypeptides which mimic the binding activity of non-peptide ligands as well as peptide or proteinaceous ligands.

## 3. SUMMARY OF THE INVENTION

The present invention encompasses a novel and efficient method or system for identifying and/or designing novel peptides and polypeptides comprising an amino acid sequence which mimics a molecular recognition site and hence the binding specificity of a naturally occurring macromolecule or of a small molecule or small region of a large molecule which functions as a ligand for a naturally occurring macromolecule. The invention also encompasses the novel peptides and polypeptides designed according to the method or system as well as conjugates containing these peptides and polypeptides. Additionally, the invention encompasses the nucleotide sequences encoding the novel peptides and polypeptides designed according to the method or system described.

The method of the invention for identifying and/or designing and preparing a novel molecular recognition units comprises:

(1) immunoselecting B cells which express an IgM immunoglobulin which binds specifically to a molecule which is or contains a molecular recognition site which is complementary to that of the molecular recognition unit desired;

(2) screening the selected B cells from step (1) to identify early B cells, i.e., those in which the sequence of deoxyribonucleotides expressed has been rearranged in only one complementarity determining region when compared to that of germline genes;

(3) determining the nucleotide sequence of or the amino acid sequence encoded by the rearranged complementarity determining region identified in step (2); and

    (4) synthesizing the desired molecular recognition unit encoded by the sequence identified in step (3) above.

According to one embodiment, B cell lymphocytes are immunoselected by (a) stimulating the production of B cell lymphocytes which are specific for an antigen or a hapten which is or contains a molecular recognition site complementary to the site desired to be mimicked; (b) immortalizing the stimulated B cell lymphocytes; and (c) screening the immortalized cells to identify B cells which secrete the desired IgM immunoglobulin which binds specifically to a molecule which is or contains a molecular recognition site complementary to that of the desired molecular recognition unit.

According to another alternative embodiment, B cell lymphocytes are "immunoselected" by screening the publicly known and/or available immortalized cell lines which produce monoclonal antibodies of the IgM isotype to identify those which react specifically with an antigen or a hapten which is or contains a molecular recognition site complementary to the desired molecular recognition unit.

The method of the invention is particularly advantageous because it can be employed to design novel peptides and polypeptides of less than about 40-45 amino acid residues that mimic the binding specificity of a very large number of molecules which contain a molecular recognition site. Indeed, the method can be employed to mimic the binding specificty of any molecule containing a molecular recognition site so long as the molecule can function as a hapten or an antigen. Thus, the molecules which can be mimicked by a peptide or polypeptide of the invention need not be peptides or polypeptides. Moreover, knowledge of the primary structure or sequence of the molecule whose binding specificity is desired to be mimicked is not required for the present method.

In addition, the method of the invention is advantageous because it provides a homogeneous supply of low molecular weight peptides and polypeptides that possess the ability to recognize and specifically bind to a desired molecular recognition site. When intended for particular in vivo uses, the peptides and polypeptides can be designed to have lower immunogenicity. On the other hand, when intended for other in vivo uses, such as for vaccines, the peptides and polypeptides can be designed to have enhanced immunogenicity.

Finally, the invention provides methods for using the novel peptides, polypeptides and conjugates for a myriad of applications in a wide range of fields including biomedicine; biological control and pest regulation; agriculture; cosmetics; environmental control and waste management; chemistry; catalysis; nutrition and food industries; military uses; and climate control.

## 4. DEFINITIONS

As used throughout the present specification, the term "Molecular Recognition Unit (hereinafter "MRU") is intended to encompass a peptide or polypeptide which comprises an amino acid sequence that mimics a molecular recognition site.

The term "molecular recognition site" is intended to encompass a domain or region of a macromolecule which recognizes and binds specifically to a small molecule or a small region of a large molecule. Examples of such molecular recognition sites include, but are not limited to: an antigen-binding site formed by hypervariable regions, also known as complementarity-determining regions ("CDRs"), of the heavy and light chains of immunoglobulins; a ligand binding site of an enzyme such as a substrate or a cofactor binding site; a ligand binding site of a receptor protein or other binding protein, etc. The term "molecular recognition site" is further intended to encompass a small molecule or a small domain of a molecule which functions as a ligand that binds specifically to a naturally occurring macromolecule. Examples of such ligands include, but are not limited to: hormones; pheromones; signal substances such as neurotransmitters, signal proteins and peptides, etc; enzyme substrates and cofactors; ligands for receptor proteins; antigens; etc.

The term "ligands" is intended to encompass a substance that specifically binds to or "fits" a molecular recognition site on a macromolecule.

The term "mimics" is intended to mean ressembles closely or simulates.

The term "early B cells" is intended to encompass B cell lymphocytes or immortalized cell lines derived from B cell lymphocytes in which the genetic information encoded in the sequence of deoxyribonucleotides expressed has been rearranged in only one complementarity determining region ("CDR") when compared to that of germline genes. As used herein, the term "rearrangement" of the sequence of deoxyribonucleotides is intended to encompass one or more of a variety of alterations including mutation, recombination, insertion and/or deletion of a nucleotide(s) from the original germline sequence of the variable region gene(s).

## 5. DETAILED DESCRIPTION OF THE INVENTION

The present invention provides a system or method for identifying and/or designing novel peptides and polypeptides termed "Molecular Recognition Units" (hereinafter "MRUs") which comprise an amino acid sequence which mimics a molecular recognition site. The molecular recognition site may be found either (a) on a naturally occurring macromolecule which binds specifically a small molecule or a small region of another macromolecule or (b) on a ligand which binds specifically to a naturally occurring macromolecule. Thus, the MRUs of the present invention mimic the binding specificity of a wide range of substances including a variety of naturally occurring macromolecules which include, but are not limited to: immunoglobulins; enzymes; receptor proteins; sensory receptors such as for taste, smell, etc.; deoxyribonucleic acid; ribonucleic acid; contractile proteins; lectins and other binding proteins such as avidin and streptavidin; etc. and a variety of ligands which include, but are not limited to: antigens; enzyme cofactors and substrates; enzyme activators and inhibitors; hormones; prostaglandins, neurohormones, growth factors; pheromones; signal substances such as neurotransmitters, signal proteins and peptides biotin pharmaceutical agents that bind to receptors or receptor proteins; transition state analogs for enzymes; etc.

Because the system of the invention entails an immunoselective method, it can advantageously be employed to design novel MRUs that mimic the binding specificity of a molecule so long as the molecule can function as a hapten or an antigen. Moreover, the system of the invention is particularly advantageous because it provides a means for rapidly designing and preparing novel peptides and polypeptides of minimum size, i.e., less than about 40-45 amino acid residues that can mimic the binding specificity of a wide variety of substances.

## 5.1. METHOD FOR IDENTIFYING AND/OR DESIGNING MRUs

According to the present invention, the method for designing MRUs comprises the following four steps:

(1) immunoselecting B cells which express an IgM immunoglobulin which binds specifically to a molecule which is or contains a molecular recognition site which is complementary to that of the MRU desired;

(2) screening the selected B cells from step (1) to identify early B cells, i.e., those in which the sequence of deoxyribonucleotides expressed has been rearranged in only one CDR when compared to that of germline genes;

(3) determining the nucleotide sequence of or the amino acid sequence encoded by the rearranged CDR identified in step (2); and

(4) synthesizing the desired MRU encoded by the sequence identified in step (3) above.

Each of these four steps is explained in detail below.

### 5.1.1. IMMUNOSELECTING B CELLS

The immunoselection step provides a means for screening the repertoire of B cell lymphocytes to identify those that produce an IgM immunoglobulin which contains a molecular recognition site whose binding specificity will be mimicked by the desired MRU being prepared. It should be noted that when the desired MRU is intended to mimic a molecular recognition site of a macromolecule, the "antigen" employed in the immunoselection step is a ligand for the macromolecule. For example, to design an MRU which mimics the binding specificity of a hormone receptor, the hormone is employed as the "antigen" to immunoselect B cells. On the other hand, when the desired MRU is intended to mimic a molecular recognition site on a ligand, the "antigen" employed in the immunoselection step is a receptor or binding protein for the ligand. For example, to design an MRU which mimics the binding specification of a hormone, the hormone receptor or an anti-hormone antibody is employed as the "antigen" to immunoselect B cells. Hereinafter, the term "Ag" is used to encompass any molecule or substance containing a molecular recognition site which is employed as an "antigen" in the immunoselection step of the present method to induce an antibody response and thus includes molecules which function as ligands and molecules which function as receptors for ligands, including but not limited to antibodies (see infra, Section 5.3.).

Any means known in the art for immunoselecting B cell lymphocytes which express an IgM immunoglobulin which binds specifically to the Ag and thus contain a molecular recognition site whose binding specificity is desired to be mimicked is useful for this step of the present method.

According to one embodiment, B cell lymphocytes are immunoselected by (a) stimulating the production of B cell lymphocytes which are specific for an Ag which is or contains a molecular recognition site complementary to the site desired to be mimicked; (b) immortalizing the stimulated B cell lymphocytes; and (c) screening the immortalized cells to identify cells which secrete the desired IgM immunoglobulin which binds specifically to a molecule which is or contains a molecular recognition site complementary to that of the desired MRU.

In an in vivo process, B cell lymphocytes are stimulated by administering the Ag, alone or in combination with an adjuvant, to an animal and the desired B cells from the spleen, lymph nodes or peripheral blood of the immunized animal are collected.

The production of an enriched population of B cells which express immunoglobulin of the IgM isotype can be enhanced using an immunization schedule in which small doses of Ag are administered for only 2-3 days or in which a single large dose of Ag is administered at 3-4 days before collection of B cells. The amount of Ag required for the in vivo immunoselection of B cells depends upon the kind of animal used and the nature of the Ag and may readily be determined by one of ordinary skill. For example, when the animal employed is a mouse or a rat, then from about 1 to about 10 $\mu$g of carbohydrate Ag; from about 10 to about 50 $\mu$g of protein Ag; about 1 $\mu$g of a bacterial or viral Ag, and larger amounts of impure Ag generally not exceeding 1000 $\mu$g is used. Additionally, if the Ag is of low molecular weight, i.e., less than about 1000 daltons, or is poorly immunogenic for any other reason, it may first be coupled to a carrier prior to administration. Suitable carriers include keyhole limpet hemocyanin, bovine serum albumin, ovalbumin, fowl immunoglobulin or any other carrier known to those of skill in the art. The in vivo process for stimulating B cells is not limited to rodent species; other species are also advantageously used as a source of B cell lymphocytes.

Alternatively, B cell lymphocytes are stimulated by an in vitro "immunization" process. This in vitro process may be particularly advantageous when the Ag of choice is in limited supply because this process may be performed using nanogram amounts of Ag rather than the microgram amounts usually used for an in vivo immunization process. Moreover, the in vitro process is useful when the Ag is labile because it requires only a few days to complete. Finally, the in vitro process is useful when the Ag is particularly toxic in vivo or cannot be administered, for example to a human, for ethical considerations.

B cell lymphocytes are stimulated in vitro by culturing spleen cells, lymph node cells, tonsillar lymphocytes or peripheral blood lymphocytes in cell culture medium with the Ag of choice for a period of about 3-5 days. When the cultured cells are obtained from a non-human animal species, it is advantageous to use thymocyte-conditioned cell culture medium. When the cultured cells are obtained for human volunteers, thymocyte-conditioned medium is not generally possible, but lymphokines may be used to stimulate proliferation of desired B cells. Additionally, in some instances, polyclonal activators of B cells such as bacterial lipopolysaccharide or mitogens such as pokeweed mitogen, phytohemagglutinin or conanavalin A can be included in the culture medium.

The B cell lymphocytes stimulated by any of the above described methods can be immortalized using any of a variety of methods known to those skilled in the art. For example, the fusion or hybridoma methods originally developed by Kohler and Milstein (reviewed by them in 1980, Sci. Amer. 243:66-74), the EBV transformation methods described by Kozbor et al. (1983, Immunol. Today 4:72-79), the EBV-hybridoma methods described by Cole et al. [in Monoclonal Antibodies and Cancer Therapy, Alan R. Liss, Inc., pp.77-96 (1985)] can be utilized to immortalize the selected B cells.

Once immortalized, the cells can be proliferated either in in vitro cell cultures or in vivo preferably as an ascites tumor or as a solid subcutaneous tumor in a compatible host animal. Alternatively, the immortalized cells can be stored frozen, for example, under liquid nitrogen and recloned periodically either in vitro or in vivo.

Generally when murine spleen cells are used, about $10^2$-$10^3$ cells are obtained at this stage of the first immunoselection step of the present method.

In order to identify B cells which secrete the desired IgM immunoglobulin, the immortalized hybridoma cells or transformed cells are cultured in vitro using cell culture medium for several days to a week or so. The supernatant is separated from the cells and tested for specificity and affinity for Ag. Any known screening technique, such as an enzyme-linked immunosorbent assay (ELISA), an immunofluorescent assay, e.g., using a fluorescence activated cell sorter (FACS), a radioimmunoassay, etc., in which the Ag is employed as the antigen is used to select B or hybridoma cells which express immunoglobulin of the desired specificity. The B or hybridoma cells must also be screened to identify those which express immunoglobulin of the IgM class. Any technique known to those of skill in the art, such as an ELISA, a radioimmunoassay, an indirect immunofluorescence assay, SDS-PAGE analysis of antibody labeled in vivo, an Ouchterlony assay, etc., can be used to determine the class of immunoglobulin produced [See, Goding, Monclonal Antibodies: Principles and Practice, 2d ed., Academic Press Inc., London 105-07 (1986); Campell, Laboratory Techniques in Biochemistry and Molecular Biology Vol. 13, Elsevier, Amsterdam, 187-93 (1984)]. Generally when murine spleen cells are used, this will represent about 10-30% of the immortalized B cells and thus about 100-300 such cells may be obtained in the first step of the invention.

According to another alternative embodiment, B cell lymphocytes can be "immunoselected" by screening the publicly known and/or available immortalized cell lines which produce monoclonal antibodies of the IgM isotype to identify those which react specifically with an Ag which is or contains a molecular recognition site complementary to the desired MRU. Such immortalized cell lines include hybridoma cell lines, EBV-transformed B lymphocyte cell lines, and cell lines formed by fusion of EBV-transformed B cells with myeloma or plasmacytoma cells. Such screening can be accomplished by computer searches of databases of published literature references and patents or by scanning the catalogues of monoclonal antibody producing cells deposited in and available from national depositories such as the American Type Culture Collection, Rockville, MD; the Agricultural Research Culture Collection (NRRL), Peoria, IL; the Collection Nationale de l'Institut Pasteur de Cultures de Microorganisms, Paris, France; to name just a few.

### 5.1.2. SCREENING FOR EARLY B CELLS

After a population of immortalized B cell lymphocytes which express IgM immunoglobulin specific for Ag of choice have been identified, the population is further screened to identify those in which the sequence of expressed deoxyribonucleotides that encode the IgM immunoglobulin has been rearranged in only a single CDR when compared to the germline sequence of deoxyribonucleotides, i.e., to identify early B cells.

According to this second step of the method of the invention, the total RNA is isolated or extracted from the immortalized B cells selected in step (1) using any technique known to those of skill in the art such as that described by Chirgwin et al. (1979, Biochem. 18:5294-99). The mRNA is separated from the rest of the RNA by affinity chromatography using either poly-U or poly-T bound to the column as described by Aviv et al. (1972, Proc. Nat'l Acad. Sci. 69:1408-13).

According to one embodiment of this step, the mRNA isolated from the selected B cells is dot blotted onto nitrocellulose filters which are then blocked to prevent subsequent non-specific binding of the labeled probe(s). Single stranded DNA oligonucleotide sequences corresponding to germline CDRs of the variable region genes from the animal from which the B cells were obtained are labeled using any known technique, including, but not limited to end labeling, nick translation, etc. These labeled DNA oligonucleotides corresponding to CDRs are particularly useful as probes. After the labeled DNA oligonucleotide probes have hybridized to the bound mRNA, the filters are washed extensively under stringent conditions (see, e.g., Maniatis, supra, pp. 382-89, incorporated herein by reference) and processed for signal development. When the label is radioactive, autoradiography permits identification of those bound fragments of mRNA which do not hybridize with the germline probes and thus produce no autoradiographic signal. These non-hybridizing mRNA fragments represent CDRs in which the germline sequence has been rearranged. The immortalized B cells in which the nucleotide sequence encoding the expressed IgM has been rearranged from germline in only one CDR can be selected using this screening step.

According to an alternate embodiment of this step of the method, the mRNA extracted from the immortalized B cells selected in step (1) is reacted with reverse transcriptase to prepare complementary single stranded DNA (cDNA). The cDNA is separated from the mRNA, digested with one or more restriction enzymes and is then separated into fragments by gel electrophoresis. The fragments are transferred to a solid substrate, such as a nitrocellulose or a nylon filter, usually by blotting and exposed to specific labeled germline DNA sequences obtained from the animal from which the B cells were obtained. The localization on the filter of the cDNA fragments which hybridize with the labeled germline DNA sequences is detected by signal development. When the label is radioactive, the localization on the filter of the hybridizing cDNA fragments is revealed by autoradiography. The cDNA fragment(s) which does(do) not hybridize with the labeled DNA probes represent(s) those in which the sequence of the CDRs have been rearranged from the germline sequence. Those immortalized B cells in which the nucleotide sequence encoding the expressed IgM has been rearranged from germline in only one CDR can be selected using this screening step.

In the practice of this alternative embodiment, the poly-A mRNA is incubated with an oligo-dT primer, reverse transcriptase and the four deoxyribonucleoside triphosphates (dNTPs). The RNA is removed from the RNA/cDNA hybrid thus formed by raising the pH of the reaction mixture. The Southern blot technique is then used to detect the specific rearranged DNA sequences in the single stranded cDNA (see, Southern, 1975, J. Mol. Biol. 98:508) as follows: The cDNA is digested with one or more restriction enzymes, mixed with marker DNA fragments of known length so that their positions can be used to estimate the lengths of the experimental cDNA fragments. The DNA mixture is then separated via gel electrophoresis and transferred by blotting to a nitrocellulose filter. Single stranded DNA corresponding to the germline sequences of the variable region genes of the animal from which the B cells were obtained are radiolabeled and used as probes to determine hybridization with the bound cDNA. Autoradiography permits identification of those bound fragments of cDNA which do not hybridize with the germline probes. These non-hybridizing cDNA fragments represent CDRs in which the germline sequence has been arranged.

According to yet another alternative embodiment of this step of the invention, polyvalent antibodies against germline variable region genes (hereinafter "anti-CDR antibodies") of the animal from which the immortalized B cells selected in step (1) were obtained are prepared using the germline CDR DNA or mRNA sequences or the amino acid sequences encoded by such germline CDRs as an immunogen in an animal different from that from which the B cells were derived. For instance, if the selected B cells were derived from a mouse, then the anti-CDR antibodies can be obtained in a rabbit or a goat. If necessary, the DNA, mRNA or amino acid sequence used an immunogen can be coupled to a carrier and administered with or without an adjuvant. The anti-CDR antibodies are then employed as the capture antibody in an immunoassay such as an ELISA, a dot blot immunoassay, sandwich immunoassays, etc. to screen either the cDNA, mRNA or IgM immunoglobulins obtained from the immortalized B cells selected in step (1). The DNA, mRNA or IgM fragments obtained from the selected cells which do not bind to the anti-DNA, anti mRNA or anti-IgM anti-CDR antibodies represent those CDRs that have been rearranged. The immortalized B cells in which the nucleotide sequence encoding the expressed ISM has been rearragned from germline in only a single CDR can be selected using this screening step.

### 5.1.3. DETERMINING THE SEQUENCE OF THE REARRANGED CDR

According to the third step of the method of the invention, the sequence of the single CDR which has been rearranged from germline and which was identified in step (2) of the method of the invention is determined. When the CDR is identified using either non-hybridizing or non-binding mRNA or cDNA fragments, the non-hybridizing or non-binding mRNA or cDNA is isolated and sequenced using conventional techniques. Using the nucleotide sequence of this single CDR which differs from germline, the amino acid sequence encoded is predicted. Alternatively, the amino acid sequence of the IgM produced by the cells having a nucleotide sequence rearragned from germline in only a single CDR can be determined using conventional amino acid sequencing methods, such as Edman degradation. The amino acid sequence encoded by such single CDR is the desired MRU of the present invention.

The mRNA is sequenced directly by a modified dideoxy-chain termination method as described by Geliebter et al., (1986, Proc. Nat'l. Acad. Sci. 83; 3371-75, the disclosure of which is incorporated herein by reference). Briefly, oligonucleotide primers specific for germline variable gene regions of the animal from which the B cells were derived are prepared, radiolabeled and incubated with poly A-mRNA in annealing buffer. The RNA-primer annealing buffer is added to an aliquot of transcription buffer containing reverse transcriptase and one dideoxyribonucleoside triphosphate (ddNTP) (i.e., ddATP, ddCTP, ddGTP or ddTTP). The reaction is stopped and the ethanol precipitate applied to a sequencing gel.

The cDNA is sequenced by known methods such as the dideoxy chain-termination method of Sanger (1977, Proc. Nat'l Acad Sci. USA 74:5463-67) or the Maxan and Gilbert (1977, Proc. Nat'l Acad Sci. USA 74:560-64) method or a combination of these methods. As is known by those of skill in the art, commercially available automated DNA sequencers are particularly useful for rapidly and efficiently determining the nucleotide sequence of even large fragments of DNA. (see, e.g., Knight, 1988, Biotech. 6:1095-96).

If the germline sequences for the animal from which the selected B cells were obtained are known, the sequence of the single CDR can be compared to verify that it differs from such sequence. In practice, the comparison is preferably performed using a computer with an appropriate software package to compare the specific DNA sequence determined with that of germline sequences which are publicly available. Examples of publicly available databases containing germline sequences include, but are not limited to the National Biomedical Research Foundation database and Intelligenetic Package (see, Smith et al., 1986, Nucleic Acids Res. 14:17-20; Dayhoff, ed., 1978, Atlas of Protein Sequence; Kabat et al., eds. 1987, Sequences of Proteins of Immunological Interest, 4th ed., U.S. Dept. of Health and Human Services, 800 pp).

When the CDR is identified using anti-amino acid anti-CDR antibodies, the amino acid sequence of the identified CDR can be directly determined using conventional methods such as Edman degradation, including the use of automated sequenators.

### 5.1.3.1. NUCLEOTIDE SEQUENCES

The invention is intended further to encompass the mRNA and DNA nucleotide sequences of the single CDRs, identified using steps (1)-(3) described above, which encode the peptide and or polypeptide MRUs. These nucleotide sequences are particularly useful to prepare not only the MRUs but also novel polypeptide and protein conjugates of the invention see, Sections 5.1.4 and 5.4, infra).

### 5.1.4. SYNTHESIZING MRUs

Once the desired MRU has been identified using steps (1)-(3) above of the present invention, the specific amino acid sequence comprising such peptide or polypeptide can be prepared using any of a number of conventional synthetic methods known to those of skill in the art. For example, the desired MRU can be prepared using any conventional method for chemical synthesis of peptides or polypeptides such as the solid phase method developed by Merrifield et al. (1963, J. Am. Chem. Soc. 85: 2149; 1982, Biochem. 21:5020; reviewed by Barany and Merrifield in Vol. 2 of The Peptides, Gross and Mierinhofer, eds., Academic Press, Inc., New York, pp. 1-284) or the liquid phase method described in Bodanszky, ed., Principles of Peptide Synthesis, Springer-Verlag pp.(1984).

Conventional recombinant DNA technique also be used to prepare the MRU. Accordingly, the DNA sequence encoding the desired MRU is inserted into a vector to form a recombinant DNA molecule or virus which is capable of replication in a compatible host cell. As would be appreciated by those of skill in the art, provided the proper replication, transcription and translation signals are correctly arranged on such recombinant DNA molecule, the DNA sequence encoding the desired MRU can be properly expressed in any of a variety of expression vectors including transformed bacterial cells or in permissive eukaryotic cells infected with a recombinant virus or recombinant plasmid carrying a eukaryotic origin of replication. Such well known recombinant techniques are described by Maniatis et al., [Molecular Cloning: A Laboratory Manual, Cold Spring Harbor Symposium, 545 pp. (1982), the disclosure of which is incorporated hereby by reference; Berger and Kimmel, eds., Guide to Molecular Cloning Techniques, Methods in Enzymology Vol. 152, Academic Press, Inc., San Diego, CA 812 pp. (1987)].

Once the desired MRU has been prepared, the specific binding of the peptide or polypeptide to the macromolecule or ligand used as Ag is tested, using any of the screening methods enumerated in Section 5.1.1, to insure that the MRU indeed binds specifically to the Ag and mimics the binding specificity of the intended molecular recognition site.

According to yet another alternate embodiment of the present invention, the above-described steps of the present method can be combined and performed using a computer with access to databases comprising the DNA and/or RNA sequences which encode monoclonal antibodies of the IgM isotype or of databases of amino acid sequences of such immunoglobulins. In practice, the publicly available databases are screened to identify those monoclonal antibodies which bind specifically to an Ag containing a molecular recognition site complementary to the desired MRU. Thus, if the desired MRU is intended to mimic the recognition site on a ligand, the databases are screened for IgM immunoglobulins specific for antibodies directed against the ligand and if the desired MRU is intended to mimic the recognition site of a receptor or binding protein, immunoglobulins specific for the ligand are screened. Examples of publicly available databases including antibody sequences include, but are not limited to the National Biomedical Research Foundation database and Intelligenetic Package (see, Smith et al., 1986, Nucleic Acids Res. 14:17-20; Dayhoff, ed., 1978, Atlas of Protein Sequence; Kabat et al., eds. 1987, Sequences of Proteins of Immunological Interest, 4th ed., U.S. Dept. of Health and Human Services, 800 pp). Once a group of IgM's specific for the chosen Ag are selected using this computer-based embodiment of the invention, the DNA sequences encoding such IgM's are compared with that of germline sequences to identify those having a variable region in which only a single CDR differs from that of the germline sequence. Such MRUs are synthesized and screened using any of the above-mentioned screening assays to verify that the thus identified MRU indeed binds specifically to the Ag and mimics the molecular recognition site intended.

The MRUs of the invention also include altered amino acid sequences in which functionally equivalent amino acid residues are substituted for residues within the sequence resulting in a silent change i.e., no adverse effect on the binding specificity of the MRU. For example, one or more amino acid residues within the sequence can be substituted by another amino acid of a similar polarity which acts as a functional equivalent, resulting in a silent alteration. Substitutes for an amino acid within the sequence may be selected from other members of the class to which the amino acid belongs. For example, the nonpolar (hydrophobic) amino acids include alanine, leucine, isoleucine, valine, proline, phenylalanine, tryptophan, and methionine. The polar neutral amino acids include glycine, serine, threonine, cysteine, tyrosine, asparagine, and glutamine. The positively charged (basic) amino acids include arginine, lysine, and histidine. The negatively charged (acidic) amino acids include aspartic and glutamic acid.

In addition, as explained in detail, infra, Section 5.4, the MRUs of the present invention are useful as components of conjugates having a first domain which mimics the specificity and binding affinity of a desired molecular recognition site and a second domain which has effector activity. When the second domain is a peptide or a polypeptide moiety, the conjugate can be prepared as a fusion protein, i.e., the product expressed by parts of two protein encoding genes ligated together so that their reading frames remain in-phase. Such fusion proteins can be prepared using any of the above-mentioned chemical or recombinant techniques either alone or in combination.

## 5.2. MRUs WITH ENHANCED AFFINITY

In certain applications, it may be desirable to prepare MRUs or conjugates of MRUs with enhanced affinity for the Ag recognized by the molecular recognition site which the MRU is designed to mimic. According to one embodiment of this mode of the invention, the MRUs prepared according to the present system can be modified according to the following procedure. Briefly, the DNA sequence encoding the particular MRU, termed "parent MRU", is chemically synthesized. Point mutations are introduced into the DNA sequence by random chemical alterations of the sequence as it is synthesized. Each of the family of mutant DNA sequences thus formed is introduced and cloned in an appropriate expression vector using conventional recobminant DNA techniques. The family of MRUs expressed is screened for binding to Ag, for example, on nitrocellulose filters. The MRU's having higher afinity than the parent MRU are identified using a competitive antigen binding assay versus the parent MRU (eee generally, Cambpell, supra, pp. 195-199 for a description of competitive assays).

According to an alternate embodiment of this mode of the invention, conjugates of MRUs with enhanced affinity for the Ag recognized by the molecular recognition site which the MRU is designed to mimic are prepared by attaching multiple copies of the MRU to a macromolecule which has multiple sites for binding the MRUs without substantially interfering with the binding specificity and/or affinity of the MRUs. As a result, such conjugates have enhanced functional affinity for the molecular recognition site in much the same way as multivalent IgM immunoglobulin molecles have higher affinity than IgG immunoglobulin molecules [see Karush, The Immunoglobulins, Litman and Goodman, eds; Plenum Press, New York pp. 85-116 (1978)].

According to yet another alternative embodiment of this mode of the invention, conjugates of MRUs with enhanced affinity for the Ag recognized by the molecular recognition site which the MRU is designed to mimic are prepared by covalently attaching multiple copies of the MRU in tandem thus forming a polymeric MRU conjugate. Such polymeric MRU conjugates can be prepared by covalently attaching a number of MRUs in tandem either directly or via a linker moiety or alternatively by ligating a number of copies of the nucleotide sequence encoding the MRU in tandem and expressing such sequence as a fusion protein in a compatible host cell. In either case, the tandem array of MRUs forming the conjugate are attached without substantially interfering with the binding specificity of the MRUs.

## 5.3. AGs

As indicated supra, Section 5.1.1, an Ag is any molecule or substance which is or contains a molecular recognition site, including molecules and substances which function as receptors for ligands, including but not limited to antibodies, enzymes, binding proteins, etc., and those which function as ligands. According to the present invention, any substance can be used as an Ag so long as it can function at least as a hapten, i.e., a molecule which binds specifically to an antibody although it may not be capable, alone, of causing the production of an antibody. When the Ag selected is a hapten, it must be coupled to a carrier moiety such as, for example, keyhole limpet hemomycanin, bovine serum albumin, ovalbumin, fowl immunoglobulin or any other known carrier etc. before being used in the method of the invention. Any antigen which is capable of inducing an antibody response can be used in the method of the present invention.

The Ag used in the practice of the present invention is chosen according to the purpose of the intended application, i.e., the binding specificity of the molecule which the desired MRU is intended to mimic. Such Ags include, but are not limited to: moieties which are more conventionally termed "antigens" such as bacterial, fungal, viral, mycoplasmal, parasitic, histocompatability or differentiation antigens; hormones; neurotransmitters; peptides that may act as both neurotransmitters and/or systemic hormones; neurohormones; pheromones; biotin; signal substances including signal peptides and proteins; growth factors; enzyme substrates and cofactors; enzyme activators and inhibitors; transition-state analogs for chemical reactions such as those which are facilitated by catalysts; contractile proteins; RNA; DNA; transport proteins; immunoglobulins, enzymes; receptors and receptor proteins; lectins and other binding proteins such as avidin and strepavidin, etc. In addition to moieties more conventionally termed antigens, including such as bacterial, fungal, viral, mycoplasmal, parasitic, histocompatibility or differentiation antigens, Table I presents a non-exhaustive list of ligands which can be used as the Ags according to the method of the present invention. Table II presents a non-exhaustive list of the kinds of macromolecules which can be used as the Ag according to the method of the present invention. In some instances, it is useful to employ an Ag which comprises a combination of more than one category of molecules containing more than one molecular recognition site. In other instances, it may be advantageous to employ an Ag which comprises a complex macromolecule which is composed of subunits which by virtue of their spatial relationships create conformational determinants.

TABLE I

EXAMPLES OF LIGANDS USEFUL AS AGs TO PREPARE MRUs

I. Hormones

Oxytocin
Vasopressin
Angiotensin
Melanocyte-stimulating hormone
Somatostatin
Thyrotropin-releasing hormone
Gonadotropin-releasing hormone
Testosterone
Estradiol
Progesterone
Cortisol
Aldosterone
Vitamin D

Gastrin
Secretin
Somatotropin
Prostaglandins
Neurotensin
Vasoactive Intestinal Peptide
Atrial Natriuretic Peptide
Thyroxine
Triiodothyronine
Insulin
Glucagon
Adrenocorticotropic hormone
Thyroid-stimulating hormone
Follicle-stimulating hormone
Luteinizing hormone
Growth hormone
Prolactin
Corticotropin-releasing hormone
Growth hormone-releasing hormone
Parathyroid hormone
Calcitonin
Chorionic gonadotropin
Chorionic somatomammotropin

II. Neurohormones

Enkephalins
Endorphins

III. Neurotransmitters

Dopamine
Norepinephirine
Glutamate
Acetylcholine
Epinephrine
Serotonin
γ-Amino Butyric Acid

IV. Growth Factors

Epidermal Growth Factor
Nerve Growth Factor
Platlet-Derived Growth Factor
Angiogenin
Fibroblast Growth Factor
Endothelial Cell Growth Factor
Granulocyte Colony Stimulating Factor
Granulocyte-Macrophage Colony Stimulating Factor
Interleukin 1
Interleukin 2
Interleukin 3
Interleukin 4
Thymopoietin
Erythropoietin
Transforming Growth Factor

V. Plant Hormones

Absciic acid
Zeatin
Indolacetic acid
Gibberellic acid

VI. Pheromones

Yeast factor a
Yeast α factor
Insect Pheromones

VII. Enzyme-Cofactors and/or Substrates

Coenzyme A
Fibrinogen
Angiotensinogen

VIII. Effectors of Enzyme Activity

Activators
Inhibitors

IX. Transition State Analogs for Enzymes

Transition-State analogs for hydrolysis of:
Esters
Carbonates
Peptides

X. Pharmaceutical Agents Which Bind to Receptors

Lovastatin
Cimetidine
Ranitidine
Bromocriptine mesylate
Mecamylamine
Isoproterenol and derivaties thereof

TABLE II

EXAMPLES OF MACROMOLECULES USEFUL AS AGs FOR PREPARING MRUs

I. Immunoglobulins

Antibodies specific for any "classical" antigens
Antibodies specific for antibodies against ligands such as those listed in Table I.

II. Receptor/Binding Proteins

Receptors for ligands such as those listed in Table I
Cell Receptors for viruses

T-Cell Receptors

α/β
γ/δ

Sensory Receptors

Taste
Smell

Lectins

Concanavalin A
Wheat Germ agglutinin
Soybean lectins
Potato lectin
Lotus seed lectin

Binding Proteins

Avidin
Streptavidin
Sperm Binding Proteins Such as ZP3
Egg Bindign Proteins

III. Enzymes

Protein enzymes
RNA enzymes
Catalytic antibodies

As used in Table II, the term "enzyme" is intended to encompass the myriad of macromolecules which are characterized by catalytic activity and specificity and which serve as catalysts of biological and/or biochemical systems. [For a general description of the activity and illustrative examples of the kinds of macromolecule intended, see Stryer, Biochemistry, 3d ed., W.H. Freeman and Co., NY, pp. 177-259 (1988); Lehninger, Principles of Biochemistry, Worth Publishers Inc., pp. 207-76 (1982) the disclsoures of which are incorporated herein by reference].

Additionally, when employed according to the method of the invention, the Ag need not be used in purified form. Thus, an Ag which may be found as part of a more complex substance such as a viral particle, a cell surface or cell surface preparation or extract, or even a tissue or organ surface and which can be presented in situ to B cell lymphocytes and still induce an antibody response can be utilized in situ according to the present invention. For example, if the Ag is a bacterial cell surface antigen, it can be used in the method of the invention either in purified form or while attached to fragments or whole bacterial cells.

5.4. CONJUGATES

The MRUs of the present invention are useful not only alone as agents that mimic the binding specificity of a variety of substances but also as components of conjugates. The conjugates of the present invention comprise a first domain which is an MRU that mimics the binding specificity and binding affinity of a desired molecular recognition site and a second domain that has effector activity. Any molecule or molecular fragment that can be attached to an MRU without destroying either the binding specificity of the MRU or the effector activity of such molecule or molecular fragment can be used as the second domain of a conjugate of the invention. In some instances, the second domain can comprise a second MRU which has effector activity. For example, an MRU which by virtue of its specificity for and binding to a given DNA sequence has a regulatory effect on such DNA sequence can be attached as an effector domain to another MRU which has specificity for a given cell, tissue or organ site. It this case, the first MRU targets the conjugate to the desired site and the second MRU exerts its regulatory activity at the desired site.

The two domains of the conjugates may be attached either directly by any covalent coupling means or via an intermediate linker moiety between the two domains. A linker is intended to include any compatible moiety having at least two reactive groups, one to react with the first binding domain and one to react with the domain having effector activity. Linkers suitable for forming conjugates of the invention include, but are not limited to the branched linkers, cleavable linkers, spacers and cleavable elements, and non-cleavable linkers described in United States Patent Application Serial No. 650,375.

When used as a component of a conjugate, the MRU targets the conjugate to the molecular recognition site for which it is specific and the effector domain exerts its activity at the target site. In those instances when the molecular recognition site for which the MRU is specific is a transition state analog for a catalytic enzyme, the MRU will have not only binding specificity, but also catalytic activity at the target site. Effector activities are intended to include biological, pharmacological and enzymic activities as well as support.

As mentioned above in Section 5.2, the effector domain can be a multivalent macromolecule with multiple sites for attachment of the MRUs such that the conjugate has enhanced affinity for the molecular recognition site for which the MRU is specific. Polymeric conjugates of MRUs are also encompassed by the MRU conjugates of the invention (see supra, Section 5.2).

Moieties which can be used as a second or effector domain of a conjugate of the invention depend upon the application for which the conjugate is intended. In some instances, the conjugate is intended for use in vivo either for a diagnostic or therapeutic purpose. In such case, the effector moiety must possess sufficient aqueous solubility so that when coupled, either directly or via a linker moiety, to an MRU, the resulting conjugate possesses sufficient aqueous solubility so that it is suitable for in vivo use. On the other hand, when the conjugate is intended for uses which do not involve administration to a human or an animal, the conjugates encompass those which are either soluble or insoluble in aqueous solutions.

When the conjugate is intended for diagnostic purposes, the MRU domain affords the ability to target the conjugate to a desired site and the effector domain provides a "reporter" moiety which allows the conjugate to be detected at the desired site. Any category of reporter moiety can be used to prepare the diagnostic conjugates of the invention including such as radioactive isotopes, radioopaque dyes, fluorogenic compounds, chemiluminescent compounds, non-radioactive paramagnetic compounds detectable using nuclear magnetic resonance spectroscopy, positron-emitting agents which can be detected using positron emission tomography, dyes detectable using conventional spectroscopy, etc. As will be readily appreciated by those of skill in the art, the diagnostic conjugates which can be employed in either in vivo or in vitro applications encompass both aqueous soluble and aqueous insoluble compositions.

When the conjugate is intended for therapeutic purposes, the MRU domain affords the ability to target the conjugate to a desired site and the effector domain provides a biologically or pharmaceutically active moiety which enables the conjugate to effect its therapeutic activity at the desired site. A large number of biologically active moieties can be used as effector domain of the the therapeutic conjugates of the invention including, for example, pharmaceutical agents such as analgesics, antibacterial agents, antifungal agents, antineoplastic and other antiproliferative agents, antiparasitic agents, antimycoplasmic agents, antiviral agents, antiinflammatory agents, antidepressants, anticoagulants, antithrombolytic agents, agents which alter membrane permeability, etc., and biologically active agents such as hormones, neurotransmitters, neurohormones, DNA, toxins and active fragments of toxins, enzymes, cytokines, lymphokines, growth factors, tumor necrosis factor, interferons, and active fragments of such biologically active agents, etc. As will be readily appreciated by those of skill in the art, the therapeutic conjugates which can be employed in either in vivo or in vitro applications encompass both aqueous soluble and aqueous insoluble compositions.

When the conjugate is intended for chemical and/or catalytic purposes, the conjugates can be classified into several different types. In one type of conjugate for these purposes, the MRU domain provides the molecular recognition and/or catalytic activity and the effector domain provides a support or matrix for the MRU domain. A large number of solid or matrix supports can be employed to prepare conjugates for these purposes. Suitable supports include, but are not limited to latex spheres, agarose beads, dextran beads, activated glass beads, polystyrene, polypropylene, rubbers, polyesters, polyamides, polymers of methacrylates, polymers of vinylchloride such as polyvinylchloride, cellulose, silica gels, and derivatives thereof, etc.

In another type of conjugate for chemical and/or catalytic purposes, the MRU domain provides the molecular recognition and/or catalytic activity and the effector domain provides a support function such that the conjugate is sufficiently soluble so that it is useful catalytically in solutions.

In yet another type of the conjugate for chemical and/or catalytic purposes, the MRU domain provides the recognition and/or catalytic activity and the effector domain provides catalytic activity which may be the same or different from the catalytic activity provided by the MRU domain. In this instance, the effector domain is an enzyme or active fragment thereof which catalyzes any of a variety of chemical reactions and the conjugate can be used catalytically in solution.

## 5.5. APPLICATIONS FOR MRUs AND CONJUGATES OF MRUs

MRUs are particularly useful in systems in which the following factors are important: molecular recognition and a high degree of binding specificity; low molecular weight and potential lower immunogenicity when administered in vivo; more rapid clearance from blood and more rapid diffusion into target organs, tissues and/or cells when administered in vivo; ability to combine with an additional effector function; ability to produce higher valent conjugates with consequent enhanced functional affinity; cost-effectiveness; etc. Thus, according to the present invention, MRUs and conjugates of MRUs are used in a wide variety of applications, including, but not limited to uses in the fields of biomedicine; biological control and pest regulation; agriculture; cosmetics, environmental control and waste management; chemistry; catalysis; nutrition and food industries; military uses; climate control; etc. The applications briefly described below are intended as illustrative examples of the uses of the MRUs and conjugates of MRUs and are in no way intended as a limitation thereon. Other applications will be readily apparent to those of skill in the art and are intended to be encompassed by this application.

The MRUs and conjugates containing MRUs of the invention are used for a wide variety of in vitro and in vivo applications in the fields of biomedicine and bioregulation or control. In these applications, the MRUs and conjugates of MRUs are employed for example, as mimetic replacements for compositions such as antibodies or immunoglobulins and fragments thereof, hormone-receptors, receptors for neurotransmitters, receptors for signal substances or proteins, and enzymes as well as mimetic replacements for antigens or immunogens, hormones, signal substances, neurotransmitters and agonists or antagonists of endogenous brain receptors for substances such as alcohol, psychoactive drugs, growth factors, pheromones, effectors of enzyme activity, gene regulatory proteins, regulators of localized cell division, etc.

Depending upon the purpose, the MRUs and conjugates the invention can be administered in vivo to animals, including humans, by a number of routes, including such as injection (e.g. intravenous, intraperitoneal, intramuscular, subcutaneous, intraauricular, intramammary, intraurethrally, etc) topical application (e.g. on afflicted areas), and by absorption through epithelial or mucocutaneous linings (e.g. ocular epithelia, oral mucosa, rectal and vaginal epithelial linings, respiratory tract linings, nasopharyngeal mucosa, intestinal mucosa, etc). Delivery to plants, insects and protists for bioregulation and/or control can be achieved by direct application to the organism, dispersion in the organism's habitat, addition to the surrounding environment or surrounding water, etc.

The MRUs and conjugates of the invention can also be employed for in vitro diagnostic and therapeutic applications by administration to cells in vitro (e.g., animal, including human cells, plant cells, protists, etc.)

In the nutrition and food industries, the MRUs and conjugates containing MRUs can be employed as mimetic replacements for any of the ligands which are recognized and specifically bound by sensory receptors which function as taste or olfactory sensors. For instance, ligands such as those which interact with receptors that mediate the taste for sweetness can be mimicked by the peptide or polypeptide MRUs of the invention. (See, Schiffman et al., The Receptors, Vol. IV, Conn, ed; Academic Press, N.Y. pp. 315-77). Such MRUs could thus serve as mimetic replacements for these ligands for sweet taste.

In the chemical industry, the MRUs and conjugates of the invention which can mimic the binding specifity of immunoglobulins, enzymes, binding proteins, or ligands for such macromolecules can be employed in a larger variety of separations, purifications and preparative methods.

In the field of catalysis, MRUs can be designed to mimic antibodies specific for transition state analogs of enzymes which catalyze any of a variety of chemical reactions.

Alternatively, MRUs can be designed to mimic the binding specificity of the active site of an enzyme, or an effector of an enzyme such as an enzyme inhibitor or an activator of an enzyme. Such MRUs can be employed to catalyze or regulate the catalysis of a variety of enzyme reactions which are useful in industrial applications.

In addition, as explained in greater detail in Section 5.4, supra, a number of different types of conjugates of MRUs can be employed as catalytic agents for reactions which are carried out either on solid supports or in solutions.

In the field of waste management, MRUs and conjugates of the invention can be employed in separations, purifications and degrative processes.

In the field of military uses and biological control, MRUs can be employed as mimetic replacements for and as novel immunogens in vaccines, as antigens in assays for toxic pathogens and toxins, as anti-toxins or antidotes, as radioprotectants, etc.

In the field of climate control, MRUs and conjugates of the invention can be employed as scavengers of pollutants which interfere with or destroy the ozone layer; as mimetics for ligands that serve as cloud seeders to increase precipitation, etc.

Finally, as indicated above, the above described applications are merely illustrative examples of the myriad uses intended for the MRUs and conjugates of the invention. Enumeration of such illustrative examples is in no way intended as a limitation either on the MRUs or the uses of the MRUs of the invention. As will be readily appreciated by those of skill in the art, the method of the present invention provides a novel way to prepare novel MRUs, which mimic the molecular recognition site of any molecule so long as it can function as a hapten or an antigen. Methods for using any such MRUs are clearly within the scope of this invention.

The following example is presented for purposes of illustration and is not intended to limit the scope of the invention in any way.

## 6. EXAMPLE: PREPARATION OF AN MRU

The following experiment illustrates the preparation of an MRU that mimics the binding specificity of an antibody that reacts with a mucin antigen associated with human adenocarcinoma according to one embodiment of the invention.

The mucin antigen is obtained in purified form from samples of human breast or colon carcinoma using a monoclonal antibody (IgG1) specifically reactive with said antigen and obtained from hybridoma cell line ACTTC No. HB8108 (hereinafter "B72.3 antibody") described in United States patent No. 4,522,918 issued to Schlom et al. (see, Nuti et al., 1982, Int. J. Cancer 29:539-45). Alternatively, the mucin antigen can be used in the form of a cell surface membrane extract obtained from samples of human breast or colon carcinoma. In either case, according to an in vivo mode of the invention, the mucin antigen is administered via an intraperitoneal route to experimental Balb/c mice for 2-3 days. On day 3, the animals are sacrificed, the spleens aseptically removed and spleen cells are immortalized using conventional fusion techniques developed by Kohler and Milstein (see review by these authors, 1980, Sci. Amer. 243:66-74).

The immortalized hybridoma cells are plated at low density, i.e., about 60% growth proportion and allowed to proliferate in in vitro cell culture for about 10-14 days in HAT medium.

The immortalized hybridoma cells obtained at about 10-14 days in cell culture are screened to identify those which have binding specificity for the mucin antigen and to identify those which express immunoglobulin of the IgM class using ELISA assays.

An aliquot of the immortalized cells identified above which express IgM immunoglobulin specific for the mucin antigen is then cultured in vitro in RPMI 1640/10% fetal calf serum and cells are harvested. Total RNA is isolated from the harvested cells after centrifugation through a CsCl cushion and poly-A-mRNA isolated using an oligo-dT cellulose column as described by Auria et al. (1972, Proc. Natl. Acad. Sci. 69:264-68).

The mRNA is then dot blotted into nitrocellulose filters which are then blocked to prevent non-specific binding when labeled germline specific single-stranded obligonucleotide probes are subsequently applied to the filters. Single stranded oligonucleotide DNA probes are prepared using the known germline sequences of murine variable region genes (see, e.g. Kabat et al., supra) and end labeled using radioactive P-32 (see, Maniatis, supra, pp. 109-124). The filters are then extensively washed under stringent conditions (see Maniatis, supra, p. 388). The washed filters are exposed to autoradiographic film to permit identification of those portions of bound mRNA which do not hybridize with the specific, radiolabeled germline DNA probes. Where no autoradiographic signal is detected, non-hybridizing mRNA fragments representing CDRs in which the germline sequence has been rearranged have been identified. Those immortalized cell lines in which the sequence encoding the expressed IgM is rearranged in only one CDR are selected using this mode of the invention.

The nucleotide sequence of the single CDR sequence identified above is determined as follows. The mRNA encoding the selected CDR is sequenced directly as described in Geliebter et al. (1986, Proc. Nat'l. Acad. Sci. 83: 3371-75).

The putative amino acid sequence encoded by the nucleotide sequence of the single CDR determined above is predicted using the conventional information of the genetic code and the amino acid sequence comprising desired MRU is chemically synthesized using the solid phase synthetic method of Merrifield (1963, J. Am. Chem. Soc. 85:2149).

The specific binding ability of the prepared MRU is erified using a competitive ELISA assay in which the mucin antigen is used as the antigen and the MRU competes with the antibody produced by immuno-selected B cells.

## Claims

1. A method for designing and preparing a molecular recognition unit, comprising:

(a) immunoselecting B cells which express an IgM immunoglobulin which binds specifically to a molecule which is or contains a molecular recognition site which is complementary to that of the molecular recognition unit desired;

(b) screening the selected B cells from step (a) to identify early B cells, i.e., those in which the sequence of deoxyribonucleotides expressed has been rearranged in only one complementarity determining region when compared to that of germline genes;

(c) determining the nucleotide sequence of or the amino acid sequence encoded of the rearranged complementarity determining region identified in step (b); and

(d) synthesizing the desired molecular recognition unit encoded by the sequence identified in step (c) above.

2. The method according to claim 1, in which the B cells are immunoselected by a method which comprises:

(a) stimulating the production of B cell lymphocytes which are specific for an antigen or a hapten which is or contains a molecular recognition site complementary to the site desired to be mimicked;

(b) immortalizing the stimulated B cell lymphocytes; and

(c) screening the immortalized cells to identify cells which secrete the desired IgM immunoglobulin which binds specifically to a molecule which is or contains a molecular recognition site complementary to that of the desired molecular recognition unit.

3. The method according to claim 1, in which the B cells are immunoselected by a method which comprises: screening publicly known and/or available immortalized cell lines which produce monoclonal antibodies of the IgM isotype to identify those which react specifically with an antigen or a hapten which is or contains a molecular recognition site complementary to the desired molecular recognition unit.

4. The method according to claim 1, in which the molecule which is or contains a molecular recognition site is selected from the group consisting of: immunoglobulins; enzymes; receptor proteins; sensory receptors; lectins and other binding proteins; deoxyribonuceic acid; ribonucleic acid; contractile proteins; antigens; enzyme cofactors and substrates; enzyme inhibitors and activators; transition state analogs of enzymes; hormones; prostaglandins, neurohormones, growth factors; pheromones; pharmaceutical agents which bind to receptors; biotin; signal substances such as neurotransmitters, signal proteins and peptides.

5. The method according to claim 1, further comprising:

(e) employing the molecular recognition unit in an assay selected from the group consisting of an enzyme-linked immunosorbent assay, an immunofluorescent assay and a radioimmunoassay to verify that the molecular recognition unit indeed binds the molecule which is or contains a molecular recognition site employed in step (a), and mimics the binding of the desired molecular recognition site.

6. A method for designing a molecular recognition unit, comprising:

(a) screening databases of either (i) deoxyribonucleotide or ribonucleotide sequences which encode monoclonal antibodies or (ii) amino acid sequences of monoclonal antibodies, to identify those sequences in which the monoclonal antibodies are of the class IgM and are specific for an antigen or a hapten which is or contains a molecular recognition site complementary to that desired to be mimicked by the molecular recognition unit;

(b) comparing the sequences identified in step (a) with germline deoxyribonucleotide sequences of the animal from which the selected monoclonal antibody sequences are derived to identify those having a variable region in which only a single complementary determining region differs from that of the germline sequences;

(c) synthesizing the amino acid sequence encoded by the single complementarity determining region identified in step (b) to form the desired molecular recognition unit; and

(d) employing the molecular recognition unit in an assay selected from the group consisting of an enzyme-linked immunosorbent assay, an immunofluorescent assay and a radioimmunoassay to verify that the molecular recognition unit indeed binds the molecule which is or contains the complementary molecular recognition site mentioned in step (a), and mimics the binding of the desired molecular recognition site.

7. A molecular recognition unit, comprising a peptide or polypeptide having an amino acid sequence which mimics a molecular recognition site and hence the binding specificity of (a) a macromolecule or (b) a small molecule or a small region of a large molecule which functions as a ligand for a naturally occurring macromolecule, prepared according to the method of claim 1.

8. A molecular recognition unit, comprising a peptide or polypeptide having an amino acid sequence which mimics a molecular recognition site and hence the binding specificity of (a) a macromolecule or (b) a small molecule or a small region of a large molecule which functions as a ligand for a naturally occurring macromolecule, prepared according to the method of claim 2.

9. A molecular recognition unit, comprising a peptide or polypeptide having an amino acid sequence which mimics a molecular recognition site and hence the binding specificity of (a) a macromolecule or (b) a small molecule or a small region of a large molecule which functions as a ligand for a naturally occurring macromolecule, prepared according to the method of claim 3.

10. A molecular recognition unit, comprising a peptide or polypeptide having an amino acid sequence which mimics a molecular recognition site and hence the binding specificity of (a) a macromolecule or (b) a small molecule or a small region of a large molecule which functions as a ligand for a naturally occurring macromolecule, prepared according to the method of claim 6.

11. The molecular recognition unit according to claim 7, in which the macromolecule is selected from the group consisting of immunoglobulins, enzymes, receptor proteins, sensory receptors, deoxyribonucleic acid, ribonucleic acid, contractile proteins, lectins and other binding proteins.

12. The molecular recognition unit according to claim 7, in which molecule which functions as a ligand is selected from the group consisting of antigens, hormones, enzyme cofactors and substrates, enzyme activators and inhibitors, transition state analogs of enzymes, prostaglandins, neurohormones, growth factors, pheromones, pharmaceutical agents which bind receptors, biotin, signal substances such as neurotransmitters, signal proteins and peptides.

13. The molecular recognition unit according to claim 8, in which the macromolecule is selected from the group consisting of immunoglobulins, enzymes, receptor proteins, sensory receptors, deoxyribonucleic acid, ribonucleic acid, contractile proteins, lectins and other binding proteins.

14. The molecular recognition unit according to claim 8, in which the molecule which functions as a ligand is selected from the group consisting of antigens, hormones, enzyme cofactors and substrates, enzyme activators and inhibitors, transition state analogs of enzymes, prostaglandins, neurohormones, growth factors, pheromones, pharmaceutical agents which bind receptors, biotin, signal substances such as neurotransmitters, signal proteins and peptides.

15. The molecular recognition unit according to claim 9, in which the macromolecule is selected from the group consisting of immunoglobulins, enzymes, receptor proteins, sensory receptors, deoxyribonucleic acid, ribonucleic acid, contractile proteins, lectins and other binding proteins.

16. The molecular recognition unit according to claim 9, in which the molecule which functions as a ligand is selected from the group consisting of antigens, hormones, enzyme cofactors and substrates, enzyme activators and inhibitors, transition state analogs of enzymes, prostaglandins, neurohormones, growth factors, pheromones, pharmaceutical agents which bind receptors, biotin, signal substances such as neurotransmitters, signal proteins and peptides.

17. The molecular recognition unit according to claim 10, in which the macromolecule is selected from the group consisting of immunoglobulins, enzymes, receptor proteins, ensory receptors, deoxyribonucleic acid, ribonucleic acid, contractile proteins, lectins and other binding proteins.

18. The molecular recognition unit according to claim 10, in which molecule which functions as a ligand is selected from the group consisting of antigens, hormones, enzyme cofactors and substrates, enzyme activators and inhibitors, transition state analogs of enzymes, prostaglandins, neurohormones, growth factors, pheromones, pharmaceutical agents which bind receptors, biotin, signal substances such as neurotransmitters, signal proteins and peptides.